(19) Europäisches Patentamt
European Patent Office
Office européen des brevets

(11) Veröffentlichungsnummer : **0 371 362 B1**

(12) **EUROPÄISCHE PATENTSCHRIFT**

(45) Veröffentlichungstag der Patentschrift : **03.02.93 Patentblatt 93/05**

(51) Int. Cl.$^5$ : **C07C 205/61,** C07C 201/12, C07C 205/47, C07C 225/00

(21) Anmeldenummer : **89121382.9**

(22) Anmeldetag : **18.11.89**

(54) **Verfahren zur Herstellung von 1-Nitroanthrachinon-2-carbonsäure.**

(30) Priorität : **30.11.88 DE 3840341**

(43) Veröffentlichungstag der Anmeldung : **06.06.90 Patentblatt 90/23**

(45) Bekanntmachung des Hinweises auf die Patenterteilung : **03.02.93 Patentblatt 93/05**

(84) Benannte Vertragsstaaten : **BE CH DE ES FR GB IT LI NL**

(56) Entgegenhaltungen :
**EP-A- 0 168 037**
**DE-A- 2 242 643**
**US-A- 1 985 232**
**ULLMANNS ENCYKLOPAEDIE DER TECHNISCHEN CHEMIE, Band 7, 4. Auflage, 1974, Seiten 585-646, Verlag Chemie, Weinheim/-Bergstrasse**
**CHEMISCHE BERICHTE, Band 101, 1968, Seiten 41-50, Verlag Chemie Gmbh, Weinheim/-Bergstrasse ; H. BREDERECK et al.:"Darstellungund Eigenschaften der Amidacetale und Aminalester"**

(73) Patentinhaber : **BASF Aktiengesellschaft**
**Carl-Bosch-Strasse 38**
**W-6700 Ludwigshafen (DE)**

(72) Erfinder : **Henkelmann, Jochem, Dr.**
**Volkerstrasse 26**
**W-6140 Bensheim (DE)**
Erfinder : **Hoch, Helmut, Dr.**
**Am Wingertsberg 14**
**W-6719 Weisenheim (DE)**
Erfinder : **Kahl, Thomas-Michael, Dr.**
**Germersheimer Strasse 146**
**W-6725 Roemerberg (DE)**
Erfinder : **Kilpper, Gerhard, Dr.**
**Am Tannenhang 1**
**W-6719 Carlsberg (DE)**
Erfinder : **Maier, Walter, Dr.**
**Bismarckstrasse 54**
**W-6700 Ludwigshafen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die vorliegende Erfindung betrifft ein neues und verbessertes Verfahren zur Herstellung von 1-Nitroanthrachinon-2-carbonsäure, sowie neue in 2-Position substituierte 1-Nitroanthrachinone.

Aus der DE-A-22 42 643, der DE-A-229 394 und Houben/Weyl, Methoden der Organischen Chemie, Band VII/3c, Seiten 255 bis 257 (1979) ist die Herstellung von 1-Nitroanthrachinon-2-carbonsäure durch Oxidation von 2-Methyl-1-nitroanthrachinon mit Chrom-(VI)-salzen in anorganischen Säuren bekannt, wobei große, nicht recyclisierbare Mengen an Chrom-(III)-salzen in den entsprechenden Säuren anfallen.

Der vorliegenden Erfindung lag daher die Aufgabe zugrunde den zuvor genannten Nachteilen abzuhelfen.

Demgemäß wurde ein neues und verbessertes Verfahren zur Herstellung von 1-Nitroanthrachinon-2-carbonsäure der Formel I

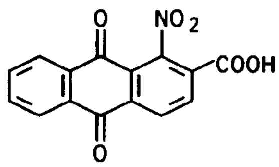

(I),

gefunden, welches dadurch gekennzeichnet ist, daß man in 2-Position substituierte 1-Nitroanthrachinone der allgemeinen Formel II

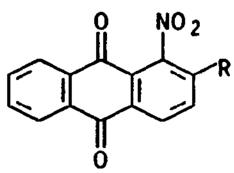

(II),

in der
R -CH=CH-$R^1$ oder -$CH_2$-CHO
bedeuten, wobei
$R^1$ für $C_1$- bis $C_5$-Dialkylamino oder ein cyclisches 5- oder 6-gliedriges Amin steht, das gegebenenfalls weitere Heteroatome enthält,
mit schwermetallfreien Oxidationsmittel behandelt, sowie die neuen Verbindungen II mit den vorgenannten Restebedeutungen.

Die 1-Nitroanthrachinon-2-carbonsäure I ist nach folgenden Methoden erhältlich:

Die Umsetzung erfolgt diskontinuierlich oder kontinuierlich durch Reaktion von den in 2-Position substituierten 1-Nitroanthrachinonen II mit schwermetallfreien Oxidationsmitteln bei erhöhten Temperaturen und Drücken zwischen 1 und 50 bar, gegebenenfalls in Gegenwart eines Starters (Initiators) nach folgender Reaktionsgleichung.

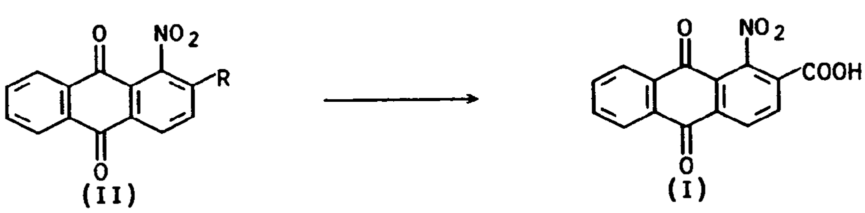

(II) (I)

Die Reaktion wird im allgemeinen bei 40 bis 200°C und 1 bis 30 bar, bevorzugt bei 50 bis 150°C und 1 bis 5 bar, besonders bevorzugt bei 60 bis 100°C und Atmosphärendruck durchgeführt.

Als schwermetallfreie Oxidationsmittel kommen anorganische Oxidationsmittel in Betracht, z.B. anorganische Säuren wie Salpetersäure und salpetrige Säure, organische Säuren wie Peroxyameisensäure, Peroxyessigsäure, Peroxytrifluoressigsäure und m-Chlorperoxybenzoesäure, Peroxide wie $H_2O_2$ und Alkylhydroperoxide und anorganische Salze, Hypohalogenide wie Natriumhypochlorid. Besonders bevorzugt sind Salpetersäure und Peroxyameisensäure.

Das Molverhältnis von schwermetallfreiem Oxidationsmittel zum in 2-Position substituierten 1-Nitroanthrachinon II beträgt von 0,7 : 1 bis 50 : 1, vorzugsweise 0,95 : 1 bis 20 : 1, besonders bevorzugt 1 : 1 bis 10 : 1.

Für die Oxidation mit Salpetersäure können Starter verwendet werden. Als Starter eignen sich $NO_x$-freisetzende Verbindungen, z.B. Nitrite wie Natriumnitrit, Kaliumnitrit, salpetrige Säure oder Stickstoffdioxid sowie Radikalinitiatoren wie Formaldehyd und Hydrochinone, bevorzugt ist Natriumnitrit.

Man verwendet 0,001 bis 10 Mol%, vorzugsweise 0,01 bis 5 Mol%, besonders bevorzugt 0,05 bis 2 Mol% des Starters, bezogen auf das in 2-Position substituierte 1-Nitroanthrachinon II. In aller Regel verwendet man 0,1 Mol%.

Es empfiehlt sich wegen der starken Wärmeentwicklung während der Reaktion die Verbindungen II, in der R für $-CH=CH-R^1$ stehen, nicht direkt in die Carbonsäure I zu überführen, sondern diese durch Hydrolyse mit einer nicht oxidierend wirkenden Mineralsäure in die Verbindung II mit R gleich $-CH_2-CHO$ zu überführen.

Die Hydrolyse wird bei Temperaturen von 40 bis 100°C, vorzugsweise bei 60 bis 100°C, besonders bevorzugt bei 80 bis 100°C unter Atmosphärendruck durchgeführt, jedoch ist die Reaktion auch bei Unter- und Überdruck möglich.

Als nicht oxidierend wirkende Mineralsäuren kommen z.B. Salzsäure, Bromwasserstoffsäure, Schwefelsäure, Phosphorsäure, bevorzugt Salzsäure und Schwefelsäure, besonders bevorzugt Salzsäure in Betracht. In aller Regel werden diese als konzentrierte wäßrige Lösungen eingesetzt, jedoch können auch inerte Lösungsmittel, z.B. Alkohole wie Methanol und Ethanol, cyclische Ether wie 1,4-Dioxan und Tetrahydrofuran mitverwendet werden.

Das Molverhältnis von nicht oxidierend wirkender Mineralsäure zu Verbindung II, in der R für $-CH=CH-R^1$ steht, beträgt von 0,9 : 1 bis 50 : 1, vorzugsweise von 0,95 : 1 bis 20 : 1, besonders bevorzugt von 1 : 1 bis 5 : 1.

Die in 2-Position substituierten 1-Nitroanthrachinone II, in der R für $-CH=CH-R^1$ steht, sind aus 2-Methyl-1-nitroanthrachinon III und einer Verbindung IV bei erhöhten Temperaturen und Drücken von 1 bis 50 bar nach folgender Reaktionsgleichung erhältlich.

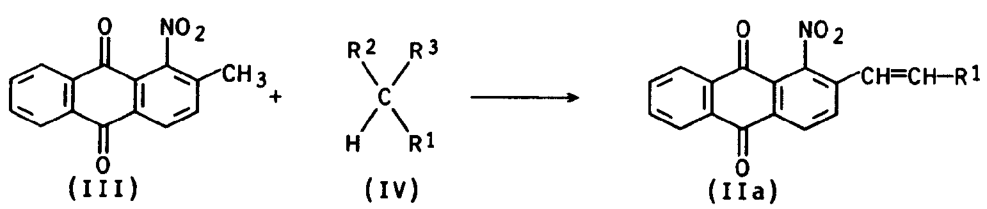

(III) (IV) (IIa)

Die Reaktion wird im allgemeinen bei Temperaturen von 60 bis 160°C, vorzugsweise 80 bis 120°C und Drücken von 1 bis 5 bar, vorzugsweise 1 bis 2 bar besonders bevorzugt bei Atmosphärendruck durchgeführt.

Die Umsetzung kann in Gegenwart von aromatischen Kohlenwasserstoffen wie Toluol, den Xylolen, Mesitylen, chlorierten aromatischen Kohlenwasserstoffen wie Chlorbenzol und den Dichlorbenzolen, cyclischen Ethern wie Tetrahydrofuran und 1,4-Dioxan, Formamiden wie N,N-Dimethylformamid, N,N-Diethylformamid, cyclischen Harnstoffen wie N,N'-Dimethylpropylenharnstoff, N,N'-Dimethylethylenharnstoff, besonders bevorzugt in N,N-Dimethylformamid durchgeführt werden. Es können auch Lösungsmittelgemische eingesetzt werden.

Die Lösungsmittelmengen sind frei wählbar, es empfiehlt sich jedoch 50 bis 500 ml pro Mol III zu verwenden.

Das Molverhältnis von IV zu III liegt im allgemeinen zwischen 0,8 : 1 und 5 : 1, vorzugsweise zwischen 1 : 1 und 2 : 1, besonders bevorzugt zwischen 1,1 : 1 und 1,5 : 1.

Die Verbindungen der Formel IV sind z.T. aus Chem. Ber. 97, 3076 bis 3080 (1964) und Chem. Ber. 101, 41 bis 50 (1968) bekannt oder können nach den dort angegebenen Verfahren erhalten werden.

Der Substituent R in der Formel II bedeutet $-CH=CH-R^1$ oder $-CH_2-CHO$.

Der Substituent $R^1$ in den Formeln IIα und IV bedeutet $C_1$- bis $C_5$-Dialkylamino, vorzugsweise $C_1$- bis $C_4$-Dialkylamino wie N,N-Dimethylamino, N,N-Diethylamino, N,N-Disopropylamino, N,N-Diisobutylamino, besonders bevorzugt $C_1$- bis $C_2$-Dialkylamino wie N,N-Dimethylamino, N,N-Diethylamino, oder ein cyclisches 5- oder 6-gliedriges Amin, das gegebenenfalls weitere Heteroatome wie Stickstoff, Sauerstoff oder Schwefel enthält wie N-Pyrrolidino, N-Piperidino, N-Morpholino, bevorzugt sind N-Pyrrolidino, N-Morpholino, besonders bevorzugt ist N-Pyrrolidino.

Die Reste $R^2$ und $R^3$ in Formel IV bedeuten unabhängig voneinander $C_1$- bis $C_5$-Alkoxy, vorzugsweise $C_1$- bis $C_4$-Alkoxy, besonders bevorzugt $C_1$- bis $C_2$-Alkoxy wie Methoxy und Ethoxy, $C_1$- bis $C_5$-Dialkylamino, bevorzugt $C_1$- bis $C_4$-Dialkylamino, besonders bevorzugt $C_1$- bis $C_2$-Dialkylamino wie Dimethylamino,

Ethylmethylamino und Diethylamino, oder gemeinsam für Alkylendioxy mit 2 bis 3 Kohlenstoffatome wie -O-$(CH_2)_2$-O- und -O-$(CH_2)_3$-O-.

Besonders bevorzugte Verbindungen II sind:

2-(2'-Dimethylaminoethenyl)-1-nitroanthrachinon
2-(2'-Diethylaminoethenyl)-1-nitroanthrachinon
2-(2'-N-pyrrolidinoethenyl)-1-nitroanthrachinon
1-Nitro-2-(2'-oxoethyl)-anthrachinon.

Besonders bevorzugte Verbindungen IV sind:

N,N-Dimethylaminodimethoxymethan
N,N-Diethylaminodimethoxymethan
Bis-(N,N-dimethylamino)-methoxymethan
Tris-(N,N-dimethylamino)-methan
N-Pyrrolidino-dimethoxymethan

1-Nitro-anthrachinon-2-carbonsäure ist u.a. Vorprodukt für Küpenfarbstoffe, wie Anthrachinonazole, Acylaminoanthrachinone und Phthaloylacridone ("Ullmanns Encyklopädie der technischen Chemie", Verlag Chemie, Weinheim 1974, Band 7, Seiten 588, 590, 609-616, 631-635 und 638).

Beispiel 1

Herstellung von 1-Nitro-anthrachinon-2-carbonsäure durch Oxidation von 1-Nitro-2-(2'-oxoethyl)-anthrachinon mit Salpetersäure:

50 g (0,17 Mol) 1-Nitro-2-(2'-oxoethyl)-anthrachinon werden in 1000 g 30 bis 65 %iger Salpetersäure suspendiert. Nach der Zugabe von 10 bis 20 mg Natriumnitrit wird das Gemisch im Verlauf einer Stunde auf 100°C erhitzt. Man rührt 5 bis 10 Stunden bei 70 bis 110°C, kühlt anschließend auf Raumtemperatur, filtriert und wäscht das gelbbraune Pulver mit Wasser bis zur neutralen Reaktion.

Das anfallende Rohprodukt wird in 1000 ml 5 %iger wäßriger Natronlauge gelöst und unter Zugabe von 25 g Natriumcarbonat zwei Stunden lang auf 50 bis 80°C erwärmt. Nach dem Filtrieren wird aus dem alkalischen Filtrat durch Ansäuern mit konzentrierter Schwefelsäure (75 %ig) bis auf pH 2 angesäuert, filtriert, mit Wasser gewaschen und getrocknet. Man erhält 1-Nitro-anthrachinon-2-carbonsäure; Fp.: 284 bis 286°C. Die Ergebnisse der Untersuchung mit verschiedenen Salpetersäurekonzentrationen sind in Tabelle 1 zusammengestellt:

Tabelle 1

| $HNO_3$ [%ig] | Zeit [h] | Temp. [°C] | Ausbeute [g] | Gehalt [%] | Reinausbeute [%] |
|---|---|---|---|---|---|
| 65 | 7 | 70-77 | 30,0 | 97 | 57 |
| 50 | 10 | 75 | 40,0 | 93 | 74 |
| 40 | 10 | 90-100 | 44,7 | 91 | 81 |
| 40 | 6,5 | 82-101 | 46,4 | 92 | 85 |
| 35 | 5 | 95-98 | 46,9 | 84 | 78 |
| 32,5 | 10 | 100-104 | 46,6 | 83 | 77 |

Das anfallende Rohprodukt kann auch durch Umkristallisation aus Ethanol oder Eisessig gereinigt werden.

Beispiel 1a

Herstellung von 1-Nitro-2-(2'-oxoethyl)-anthrachinon

100 g (0,31 Mol) 2-(2'-Dimethylaminoethenyl)-1-nitro-anthrachinon wird mit 200 g konzentrierter Salzsäure (38 %ig) und 600 g Wasser 2 h unter Rückfluß erhitzt. Nach dem Abkühlen filtriert man die ausgefallenen Kristalle ab, wäscht mit Wasser bis das Filtrat neutral ist und trocknet bei 70°C. Man erhält 90,5 g (98,7 %) 1-Nitro-2-(2'-oxoethyl)-anthrachinon; Fp: 192°C.

Beispiel 2

Herstellung von 1-Nitro-anthrachinon-2-carbonsäure durch Oxidation von 2-(2′-Dimethylaminoethyl)-1-nitro-anthrachinon mit Salpetersäure.

50 g (0,16 Mol) 2-(2′-Dimethylaminoethenyl)-1-nitro-anthrachinon werden in 50 g konzentrierter Salpetersäure (65 %ig) und 390 g Wasser suspendiert und 2 Stunden unter Rückfluß erhitzt. Nach dem Abkühlen auf Raumtemperatur fügt man 500 bis 1000 g konzentrierte (65 %ige) Salpetersäure und 10 bis 20mg Natriumnitrit hinzu und erhitzt im Verlauf einer Stunde auf 100°C. Man rührt 7 bis 10 h bei 90 bis 100°C, kühlt wieder auf Raumtemperatur ab, filtriert und wäscht das hellbraune Pulver mit Wasser bis zur neutralen Reaktion.

Das anfallende Rohprodukt wird in 1000 ml 5 %iger Natronlauge gelöst und unter Zugabe von 25 g Natriumcarbonat zwei Stunden lang auf 50 bis 80°C erwärmt. Nach dem Filtrieren wird aus dem alkalischen Filtrat durch Ansäuern mit konzentrierter Schwefelsäure (75 %ig) bis auf pH 2 angesäuert, filtriert, mit Wasser gewaschen und getrocknet. Man erhält 1-Nitro-anthrachinon-2-carbonsäure; Fp.: 284 bis 286°C.

Die Ergebnisse mit unterschiedlichen Salpetersäuremengen sind in Tabelle 2 zusammengestellt:

Tabelle 2

| Zeit [h] | $HNO_3$ 65 %ig [g] | Ausbeute [g] | Gehalt [%] | Reinausbeute [%] |
|---|---|---|---|---|
| 10 | 580 | 42 | 90 | 82 |
| 7 | 800 | 40 | 84 | 72 |
| 2 | 1000 | 40 | 80 | 69 |

Das anfallende Rohprodukt kann auch durch Umkristallisation aus Ethanol oder Eisessig gereinigt werden.

Beispiel 2a

Herstellung von 2-(2′-Aminoethenyl)-1-nitro-anthrachinone

267,0 g (1,0 Mol) 2-Methyl-1-nitro-anthrachinon und 1 bis 2 Mol des entsprechenden Amino-dimethoxymethans in 1 000 g N,N-Dimethylformamid werden 6 bis 8 h zum Rückfluß erhitzt. Anschließend wird bis zu einer Sumpftemperatur bis zu 110°C destilliert. Nach Abkühlen wird der tiefviolette Niederschlag abgesaugt, mit wenig N,N-Dimethylformamid und Wasser gewaschen und getrocknet. Man erhält nach Einengen des Filtrats und Umkristallisieren des Rückstandes aus Chlorbenzol die aus Tabelle 3 ersichtlichen Ergebnisse.

Tabelle 3

| 2-(2'-Aminoethenyl)-1-nitro-anthrachinon | Fp: [°C] | Ausbeute [%] |
|---|---|---|
| 2-(2'-Dimethylaminoethenyl)-1-nitro-anthrachinon | 268-272 unter Zers. | 95 |
| 2-(2'-Diethylaminoethenyl)-1-nitro-anthrachinon | 235-241 unter Zers. | 90 |
| 2-(2'-Diisobutylaminoethenyl)-1-nitro-anthrachinon | 177 | 87 |
| 1-Nitro-2-(2'-N-pyrrolidinoethenyl)-anthrachinon | 235-239 unter Zers. | 90 |

Beispiel 2b

Herstellung von 2-(2'-Diethylaminoethenyl)-1-nitro-anthrachinon

42,0 g (0,16 Mol) 2-Methyl-1-nitro-anthrachinon (0,16 Mol), 46,2 g (0,32 Mol) Diethylaminodimethoxymethan (0,32 Mol) in 150 g Diethylformamid werden 7 h zum Rückfluß erhitzt. Nach dem Abdestillieren aller flüchtigen Bestandteile wird der Rückstand in Wasser aufgeschlämmt, abgesaugt und im Vakuum getrocknet. Man erhält 49,6 g (90,0 %) 2-(2'-Diethylaminoethenyl)-1-nitroanthrachinon; Fp.: 235 bis 241°C (unter Zersetzung).

Beispiel 3

Herstellung von 1-Nitro-anthrachinon-2-carbonsäure durch Oxidation von 2-(2'-Dimethylaminoethenyl)-1-nitroanthrachinon mit Wasserstoffperoxid in Ameisensäure/Essigsäure

Zu einer Lösung von 10 g (0.031 mMol) 2-(2'-Dimethylaminoethenyl)-1-nitro-anthrachinon in 100 ml Ameisensäure bzw. 100 ml Essigsäure gibt man die 5- bis 15-fache molare Menge einer 30 %igen Wasserstoffperoxid-Lösung in Wasser. Nach 2 bis 10 h Rühren wird die entstandene Suspension in 200 ml Wasser gegeben, abfiltriert, neutral gewaschen und getrocknet. Die Reinigung erfolgt analog Beispiel 2.

Die Ergebnisse bei der Verwendung verschiedener Reaktionstemperaturen sind in Tabelle 4 zusammengestellt:

Tabelle 4

| | T [°C ] | Zeit [h] | $H_2O_2$ | Ausbeute [g] | Gehalt [%] | Reinausbeute [%] |
|---|---|---|---|---|---|---|
| Ameisensäure | 28°C | 10 | 0,31 Mol | 8,6 | 84 | 78 |
| Ameisensäure | 28-50°C | 4 | 0,31 Mol | 9,2 | 89 | 89 |
| Ameisensäure | 50°C | 4 | 0,31 Mol | 9,0 | 89 | 87 |
| Ameisensäure | 80°C | 3 | 0,16 Mol | 8,8 | 49 | 47 |
| Ameisensäure | 90°C | 7 | 0,31 Mol | 8,7 | 76 | 72 |
| Essigsäure | 50°C | 6 | 0,31 Mol | 9,0 | 71 | 69 |

## Patentansprüche

1. Verfahren zur Herstellung von 1-Nitroanthrachinon-2-carbonsäure der Formel I

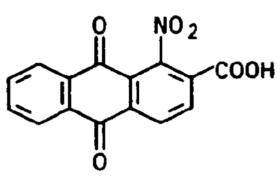

(I),

dadurch gekennzeichnet, daß man in 2-Position substituierte 1-Nitroanthrachinone der allgemeinen Formel II

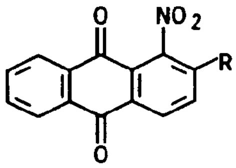

(II),

in der
R $-CH=CH-R^1$ oder $-CH_2-CHO$
bedeuten, wobei
$R^1$ für $C_1$- bis $C_5$-Dialkylamino oder ein cyclisches 5- oder 6-gliedriges Amin steht, das gegebenenfalls weitere Heteroatome enthält,
mit schwermetallfreien Oxidationsmitteln behandelt.

2. Verfahren nach Anspruch 1, dadurch gekennzeichnet, daß man die in 2-Position substituierten 1-Nitroanthrachinone der allgemeinen Formel II

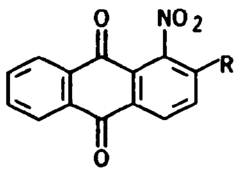

(II)

für den Fall, daß R $-CH=CH-R^1$ bedeutet, durch Umsetzung von 2-Methyl-1-nitroanthrachinon der Formel III

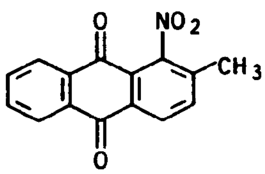

(III),

mit Verbindungen der allgemeinen Formel IV

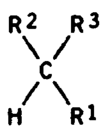

(IV),

in der $R^1$ die in Anspruch 1 genannten Bedeutungen besitzt und $R^2$, $R^3$ unabhängig voneinander für Alkoxy, Dialkylamino oder gemeinsam für Alkylendioxy stehen, erhält und für den Fall, daß R $-CH_2-CHO$ bedeutet, die zuvor erhaltenen Verbindungen mit R gleich $-CH=CH-R^1$ mit einer nicht oxidierend wirkenden Mineralsäure behandelt.

3. In 2-Position substituierte 1-Nitroanthrachinone der allgemeinen Formel II

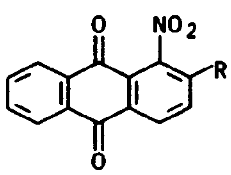

(II),

in der

R $-CH=CH-R^1$ oder $-CH_2-CHO$
bedeutet, wobei
$R^1$ für $C_1$- bis $C_5$-Dialkylamino oder ein cyclisches 5- oder 6-gliedriges Amin steht, das gegebenenfalls weitere Heteroatome enthält.

## Claims

1. A process for preparing 1-nitroanthraquinone-2-carboxylic acid of the formula I

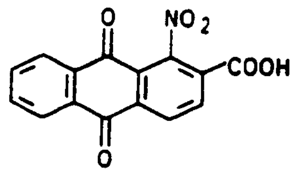

(I),

which comprises treating a 2-substituted 1-nitroanthraquinone of the general formula II

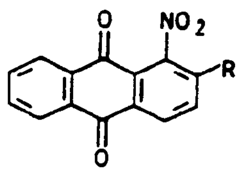

(II),

where R is $-CH=CH-R^1$ or $-CH_2-CHO$, where $R^1$ is $C_1$-$C_5$-dialkylamino or a cyclic 5- or 6-membered amine which may contain further hetero atoms, with an oxidizing agent free of heavy metal.

2. A process as claimed in claim 1, wherein the 2-substituted 1-nitroanthraquinone of the general formula II

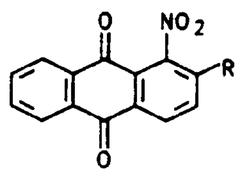

(II)

is obtained if R is $-CH=CH-R^1$ by reacting 2-methyl-1-nitroanthraquinone of the formula III

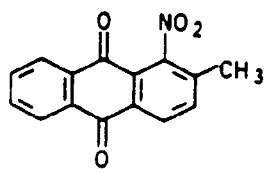

(III),

with a compound of the general formula IV

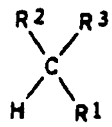

where $R^1$ is as defined in claim 1 and $R^2$ and $R^3$ are singly, and independently of each other, alkoxy or dialkylamino or together alkylenedioxy, and if R is $-CH_2-CHO$ by treating the previously obtained compound where R is $-CH=CH-R^1$ with a non-oxidizing mineral acid.

3. A 2-substituted 1-nitroanthraquinone of the general formula II

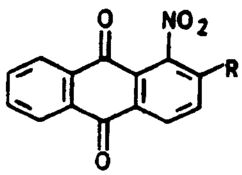

(II),

where R is $-CH=CH-R^1$ or $-CH_2-CHO$, where $R^1$ is $C_1$-$C_5$-dialkylamino or a cyclic 5- or 6-membered amine which may contain further hetero atoms.

## Revendications

1. Procédé de préparation de l'acide 1-nitroanthraquinone-2-carboxylique de la formule I

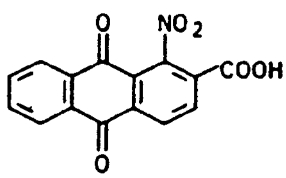

(I),

caractérisé en ce que l'on traite des 1-nitroanthraquinones de la formule générale II

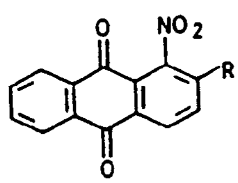

(II),

dans laquelle
R représente un radical $-CH=CH-R^1$ ou $-CH_2-CHO$
dans lequel
$R^1$ représente un groupe dialkylamino en $C_1$ à $C_5$, ou une amine cyclique pentagonale ou hexagonale, qui contient éventuellement d'autres hétéroatomes,
par des agents d'oxydation dépourvus de métaux lourds.

2. Procédé suivant la revendication 1, caractérisé en ce que, dans le cas où R représente le radical $-CH=CH-R^1$, on obtient les 1-nitroanthraquinones substituées en position 2 de la formule générale II

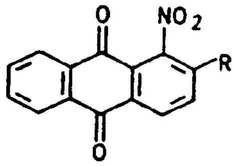

(II)

par la réaction de la 2-méthyl-1-nitroanthraquinone de la formule III

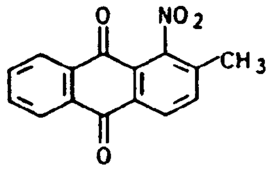

(III),

avec des composés de la formule IV

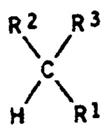

(IV),

dans laquelle $R^1$ possède les significations qui lui ont été attribuées dans la revendication 1 et $R^2$ et $R^3$ représentent, indépendamment l'un de l'autre, des radicaux alcoxy, dialkylamino, ou représentent ensemble un radical alkylènedioxy et, dans le cas où R représente le radical $-CH_2-CHO$, on traite les composés précédemment obtenus où R désigne $-CH=CH-R^1$ par un acide minéral dépourvu d'activité oxydante.

3. 1-Nitroanthraquinones substituées en poistion 2 de la formule générale II

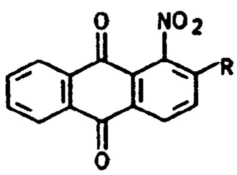

(II),

dans laquelle
R représente un radical $-CH=CH-R^1$ ou $-CH_2-CHO$
dans lequel
$R^1$ représente un groupe dialkylamino en $C_1$ à $C_5$, ou une amine cyclique pentagonale ou hexagonale, qui contient éventuellement d'autres hétéroatomes.